# EUROPEAN PATENT APPLICATION

(11) **EP 1 911 835 A1**
(43) Date of publication of application: **16.04.2008**
(21) Application number: 05795556.9
(22) Date of filing: 22.09.2005
(51) Int. Cl.: C12N 5/00, C12N 5/06, C12M 3/00, A61P 31/18, A61P 35/00

(54) **A PURIFICATION AND CULTIVATION METHOD OF OCEAN SPONGE PLURIPOTENT STEM CELL**

(30) Priority: 05.02.2005 CN 200510045852
(71) Applicant: Dalian Institute of Chemical Physics, Chinese Academy of Sciences, Dalian City, Liaoning Province 116023 (CN)
(72) Inventor: ZHANG, Wei, Dalian, Liaoning 116023 (CN); SUN, Liming, Dalian, Liaoning 116023 (CN); JIN, Meifang, Dalian, Liaoning 116023 (CN); YU, Xingju, Dalian, Liaoning 116023 (CN)
(74) Representative: Stuart, Ian Alexander
(86) International application number: PCT/CN2005/001540
(87) International publication number: WO 2006/081722

(57) **Abstract**

This invention related to field of marine biotechnology. It is to disclose a purification and cultivation method of multipotential stem cell of marine sponge. The mix sponge cells were progressively treated by differential centrifugation, differential aggregation, differential adherence and density gradient centrifugation, purification carries out according to the properties of sponge cells (size, aggregation ability, adhering pattern and density). The activity of purified archaeocytes was over 95%. Growth factors and differentiation- restraining factors could promote archaeocytes to grow and their differentiation was controlled. This invention could produce bioactive compounds through large-scale archaeocytes culture in vitro, for the biosynthesis of bioactive compounds.

## Description

### FIELD OF THE INVENTION

The present invention is in the field of marine biotechnology, involving in vitro cell culture of marine invertebrate and production technology of marine drugs. It is a method of purification of marine sponge stem cell and in vitro culture. The present invention could be applied in the proliferation culture of marine sponge cells, also in the production of sponge bioactive compounds and biosilica materials.

### BACKGROUND OF THE INVENTION

Marine sponges are the lowest multi-cellular invertebrate animals, which feed by filtrating sea water. A large number of novel marine bioactive compounds, with strong anti-tumour, anti-inflammation and anti-HIV activities, have been isolated from marine sponges; many of these compounds are of potential clinical value. From published data, new bioactive compounds from marine sponge account for 40% of the total found from marine sources in 2000, and 36.3%,38.2 and 32.6% respectively in 2002,2003,2004. More than 10% of the compounds from marine sponge are cytotoxic, regarding the possibilities from other sources: 2% for other marine soucrces, and less than 1% for terrestrial propagation and microorganism. Among so many compounds, Ara-A and Avarol were used in clinical practice, some other compounds were studied in the stage of clinical or pre-clinical researches. The content of most bioactive compounds in marine sponge is very low. The quantity of extractable sponge biomass available from the ocean usually cannot meet the demand for large scale research and clinical development of these compounds. The conditions for the field culture of marine sponges are complex and it's hard to improve the production by regulations. So it's difficult to produce the bioactive compounds in a large amount. And the same time, most of these compounds are complex in structure, which cause the high cost but low output of the chemical synthesis. And the environmental pollution will be another problem caused by chemical synthesis. That's the reason for the facts that although lots of bioactive compounds have been discovered from the marine sponges, but just a few come into the market. Most of these compounds share the so-called "Programming death of pharmaceutical studies", i.e. the continous clinic studies are ceased due to the lack of the economical biotechnological pathway to supply enough compound sources. So the study and establishment of a platform for marine sponge products to obtain the controlled, high performance production in a large amount is the key point to break through the bottleneck of the R&D and commercialization of the sponge-derived bioactive compounds. The studies on in vitro bioreactor culture of marine sponge cells draw more and more attentions internationally, and it becomes one of the most hopeful approach to solve the supply problem of the sponge biomass. But there are still some problems to be solved before the large scale bioreactor production. First of all, sponges have their own morphological characters, i.e. there is abundant canal system inside the sponge, especially inside some small size sponges, such as *Hymeniacidon perleve.* The porous body of sponges brings the trouble to locate the definite place as the cell source. So in the primary cultures, the whole body is dissociated and the mixed sponge cell population is obtained. Then the mixed cells will be cultured directly or after briefly separation.

Sponges are multi-cellular animals, composing archaeocyte, collencyte, sclerocyte, pinacocyte, choanocyte, myocyte, poroctye, gray cell, spherulous cell, bacteriocyte, cystencyte et al. Among them, archaeocytes are multipotential stem cell, and have the ability to proliferation and differentiation. Archaeocytes could differentiate into other component cells such as collencytes, sclercotyes, pinacocyte and choanocytes. The proliferation and differentiation ability of these cells weaken or lose.

As in vitro sponge cell culture is a challenging project, selecting proliferation-potential archaeocytes to culture in vitro is critical for establishment of sponge cell line and large-scale bioreactor culture to produce bioactive compounds. Separation and purification of archaeocytes is prerequisite of the above work. Since there is little research about the specific membrane protein of archaeocytes and marked difference exists between sponges from different living conditions or between different species, the specific marker of archaeocyte as well as the routined, common and specified protocol for archaeocyte separation is unknown. So, archaeocytes could not be separated by some standard, specific methods, such as FCM (Flow cytometry). Most cultures in vitro used mixed sponge cells. Density gradient centrifugation is always used in archaeocytes fractionation, but this method can't highly purify archaeocytes.

### SUMMARY OF THE INVENTION

To overcome the limitation of other sponge cell types without proliferation capability coexist in the arcaeocytes culture system, this invention is to raise a method to purify and culture archaeocytes (multipotential stem cell of sponge), this invention could produce bioactive compounds through large-scale archaeocytes culture in vitro.

In order to realize the purposes above-mentioned, the technical scheme of this invention is as follows:

According to the difference of size and adhesive ability, the mixed sponge cells are treated in turn by differential centrifugation, differential aggregation, differential adhesion and density gradient centrifugation. Purification carries out according to the properties of sponge cells of size, adhesion and etc.

### 1) Pretreatment (paration of the mixed sponge cells)

The marine sponge specimens were immediately transferred to the laboratory or kept in an aquarium for 1-2 weeks before use. Healthy sponge specimens were soaked in sterile NSW (natural seawater) supplemented with gentamicin (200~800µg/ml). Sponge specimens were cut into small pieces (1~3mm³) after the remove of the impurities and rinsed in sterile CMFSW (Ca²⁺/Mg²⁺-free seawater) for 3-5 times, 2-3hours each time. The small sponge pieces were dissociated and the resultant cell suspension was filtered and centrifuged at 1000~3000rpm for 10~20min, the pellets were resuspended in sterile CMFSW with 1-2 mM EDTA to prevent the cells aggregating. Hence, mixed sponge cells were prepared.

### 2) Purification of multipotential stem cell of marine sponge

### 2.1 Differential centrifugation

Adjust the density of the mixed sponge cells of the step 1) with CMFSW to a fitful value, then centrifugated at 300∼600 rpm for 10∼20min, discarded the supernatant, the pellets were mostly big sponge cells including archaeocytes.

### 2.2 Differential aggregation

The sponge cells obtained by differential centrifugation as above step 2.1 were washed with CMFSW to discard EDTA, and resuspended in CMFSW at a density of 5-10×10⁷/ml. Then the sponge cells were cultured at 18-25°C for 8-12 h allowing the archaeocytes to agglomerate. The aggregates were collected by decanting the non-aggregating suspension cells.

### 2.3 Differential adhesion

Collected aggregates were cultured in ASW (Artificial Sea Water) to adhere to the substrate. The pinacocytes and collencytes in the aggregates started to adhere to the bottom of culture flasks or dishes. These cells then flattened and spread actively, once the cells confluence covered 70-80% of the culture area, the ASW was washed off with CMFSW and the archaeocytes were dissociated with CMFSW containing 2~10 mM EDTA. The treatment of EDTA was controlled under light microscope for 5-10 minutes at 18-22°C. Based on the adhering pattern of non-archaeocyte, this step removed most pinacocytes and collencytes from the aggregates. The detached archaeocytes cells were completely dissociated in CMFSW-E for the final step of density gradient centrifugation.

### 2.4 Density gradient centrifugation

2.4.1 The sponge cells harvested at step 2.3 were adjusted to a concentration of 5-10×10⁷/ml for density gradient centrifugation.
2.4.2 Prepare the density gradient solution
2.4.3 The cells preliminarily dissociated were density gradient centrifuged after the concentration was adjusted.

Continuous gradients were developed by gentle layering of 1-10 ml successively less-dense solutions. Then, 1-5 ml dissociated enriched archaeocyte suspension with a total cell number 2~20×10⁷ was applied on the top of the gradient. After centrifugation, the archaeocyte fraction were collected and washed with CMFSW by centrifugation at 500~1500rpm for 5-20min to remove gradient solution. This washing process was carried out for 2-4 times, thus the harvest archaecytes, i.e. multipotential stem cell, had been separated from other cell types.

By the way, in the step 2.3, it's better to dissociate outlayer archaeocytes of the cell aggregates by CMFSW with 2-5mM EDTA.

Density gradient described in step 2.4 was prepared as follows: A Ficoll-Vrografin density gradient protocol was developed to purify the archaeocytes. Solutions of 3~9% Ficoll (F) and 34~38% Vrografin (V), which diluted from 76%, were prepared in sterile CMFSW at a density of 1.0276∼1.1486 g/ml and 1.1379∼1.1486 g/ml at 16~20°C, respectively. Continuous Ficoll-Vrografin (FV) gradients with densities of 1.04/1.05/1.06/1.07/1.08/1.09/1.10 g/ml were developed.

Density gradient described in step 2.4 could also be prepared with sucrose as follows: sucrose solutions were preared in sterile CMFSW at a concentration of 12~24%, 24~36%, 36~48%, 48~52%, 58~60%. Continuous sucrose gradients were developed by gentle layering successively less-dense sucrose solutions. The volume of each gradient solution is 1-2 ml when using 15ml tube, and 1~2 ml dissociated enriched archaeocyte suspension was applied on the top of the gradient. The volume of each gradient solution is 5~10 ml when using 50ml tube, and 3-5 ml dissociated enriched archaeocyte suspension was applied, the cells were totally 1~2×10⁸.

Sponge cell culture in vitro and metabolism detection were added in this invention. The purified archaeocytes were cultured in a density of 2×10⁶~3×10⁷ cells/ml with ASW at 18~25°C, at rest or rotating at 20~40rpm. The culture media was changed by 50-70 percent every 1-2days. The total cell number and PCNA positive cell number were counted. At the same time, mixed sponge cell culture was also carried out as control. The component of the media used for cell culture is. ASW with intercellular substance of the sponge, cell growth factors and differentiation-controlling factors. The cells could be cultured in light or dark according to the sponge species.

The described intercellular substance of sponge is prepared as follows: Freshly collected sponges were dipped in CMFSW in ration of 1/10~1/20 (w/v). The CMFSW was changed by 80-100percent every 2-4hours, totally 4-6times. All the lixivium collected and filter-sterilized. The extracted intercellular substance was added to the cell culture system by 25-75persent.

The described cell growth promoting factors are: 5-20mg/ml soluble cholesterol, 5-20µg/ml hydrocortisone, 5-10µg/ml insulin ,5-10µg/ml transferrin, 6-10×10⁻⁵g/L Na₂SeO₃, 15-50µg/ml phytohaemagglutinin (PHA), 100-200µg/ml DTT(1,4-Dithiothreitol), 2-8×10⁻⁴g/L glutathione

The described differentiation-controlling factors using any one selected from the group consisting of 5-50µM/ml hydroxyacrbamide, 5-20µM/ml retinoic acid, and mixtures thereof.

Compared with other similar invention in prior art, this invention has some strongpoint:
1) This invention developed a novel multi-method coupling four-step protocol for the purification of multipotential stem cell, archaeocytes, from a marine sponge. The purified archaeocytes could be used as seed cells in culture in vitro. Compared with the ordinary methods which use density gradient centrifugation directly to separate archaeocytes, this invention used differential centrifugation, differential aggregation and differential adhesion before density gradient centrifugation, these three steps could raise the purity(more than 80%) and recycling rate (50-60%) of separation process, and the activity of the purified archaeocytes are more than 95%.Thus this invention could highly purify archaeocytes, and will make it possible for the future research of the archaeocyte biology and culture in vitro.
2) The archaeocytes purified with the method described in this invention could be cultured in vitro. The added cell growth and differentiation-controlling factors could promote archaeocytes to proliferate.
3) The archaeocytes purified with the method described in this invention could be cultured large scale in vitro, this system could be used for bioactive compounds production.

### BRIEF DESCRITION OF THE ATTACHED DRAWINGS

Fig.1-1 Sponge cells before differential centrifugation (Mixed sponge cells, ×400)).
Fig.1-2 Immunocytochemical assay to detect BrdU positive cells before differential centrifugation (BrdU positive cells in mixed sponge cells (×400))
Fig.1-3 Immunocytochemical assay to detect PCNA positive cells before differential centrifugation (PCNA positive cells in mixed sponge cells (×400)).
Fig.1-4 Sponge cells harvested by differential centrifugation (sponge cells harvested by centrifugation at 500rpm for15min, ×400)).
Fig. 1-5 Immunocytochemical assay to detect BrdU positive cells after differential centrifugation (BrdU positive cells in sponge cells harvested by centrifugation at 500rpm for 15min (×400)).
Fig.1-6 Immunocytochemical assay to detect PCNA positive cells after differential centrifugation (PCNA positive cells in sponge cells harvested by centrifugation at 500rpm for 15min (×400)).
Fig.2-1 Differential aggregation of sponge cells (Sponge cell aggregation quickly formed in30min (×40)).
Fig.2-2 Differential adhesion of sponge cells (cultured for 2 hours, Sponge cell aggregation began adhere to the bottom, collencytes began migrate outwards (×40)).
Fig.2-3 Differential adhesion of sponge cells (cultured for 4 hours, collencytes went on migrate outwards, the sponge cell aggregation began spread out (×40))
Fig.2-4 Differential adhesion of sponge cells (cultured for 6 hours, the sponge cell aggregation went on spread out and became confluence introduced by collencytes (×250)).
Fig.2-5 Differential adhesion of sponge cells (cultured for 8 hours, the sponge cell aggregation reached a maximum confluence (×400))
Fig.2-6 Differential adhesion of sponge cells (archaeocytes were detached from the collencyte layer, the pinacocyte and collencytes were still adhere to the bottom (×400)).
Fig.2-7 Differential aggregation of sponge cells (sponge cells harvested by differential aggregation, mixed with some collencytes and other small cells in small amounts (×400)).
Fig.2-8 Differential aggregation of sponge cells (Immunocytochemical assay to detect BrdU positive cells in the sponge cells harvested by differential aggregation (×400)).
Fig.2-9 Differential aggregation of sponge cells (Immunocytochemical assay to detect PCNA positive cells in the sponge cells harvested by differential aggregation (×400)).
Fig.2-10 Differential adherence of sponge cells (sponge cells harvest by differential adherence (×400)) .
Fig.2-11 Differential adherence of sponge cells (Immunocytochemical assay to detect BrdU positive cells in the sponge cells harvested by differential adherence (x400)) .
Fig.2-12 Differential adherence of sponge cells (immunocytochemical assay to detect PCNA positive cells in the sponge cells harvested by differential adherence (×400)).
Fig.3-1 Density gradient centrifugation to purify archaeocytes (density gradient produced by Ficll and Vrografin) .
Fig.3-2 Distribution of the sponge cells after the gradient.
Fig.4-1 Sponge cells harvested by density gradient centrifugation (archaeocytes were at layer C5 (×400)).
Fig.4-2 Immunocytochemical assay to detect BrdU positive cells after the whole purifying process(BrdU positive cells in the purified archaeocytes (×400)).
Fig.4-3 Immunocytochemical assay to detect PCNA positive cells after the whole purifying process(PCNA positive cells in the purified archaeocytes (×400)).
Fig.5 Percentages of archaeocytes and BrdU-positive and PCNA positive cells in sponge cell samples obtained at each step of the combined separation method (MC, mixed cells; DC, sponge cells obtained by differential centrifugation; DA, differential aggregation; DAD, differential adherence; DGC density gradient centrifugation).
Fig.6 Effect of incubation time on the incorporation of BrdU into the archaeocytes.
Fig.7 Dynamic curve of cell growth in terms of MTT activity during primary culture in vitro between the purified archaeocytes and mixed cell population (Cell growth is monitored by a MTT assay, MTT value is relevant with the cell number and activity, relative growth index was defined as the MTT value on day N dividing by the MTT value on 1^{st} day).
Fig. 8 Dynamic curve of cell growth in terms of total cell number during primary in vitro culture between the purified archaeocytes and mixed cell population i.e. the cell growth is described by their number changes.
Fig.9-1 Cell samples of marine sponge collected from South China Sea harvested by each step of the method described in the invention (mixed sponge cells).
Fig.9-2 Cell samples of marine sponge collected from South China Sea harvested by each step of the method described in the invention (sponge cells harvested after centrifugation at 500rpm for 10min).
Fig.9-3 Cell samples of marine sponge collected from South China Sea harvested by each step of the method described in the invention (sponge cell aggregation harvested after after differential aggregation)
Fig.9-4 Cell samples of marine sponge collected from South China Sea harvested by each step of the method described in the invention (archaeocytes were detached from the pinacocyte and collencyte layer) .
Fig.9-5 Cell samples of marine sponge collected from South China Sea harvested by each step of the method described in the invention (a kind of cortex cell harvested by density gradient centrifugation) .
Fig.9-6 Cell samples of marine sponge collected from South China Sea harvested by each step of the method described in the invention (archaeocytes harvested by density gradient centrifugation) .
Fig.10-1 Distribution of the cell samples of marine sponge collected from South China Sea after velocity sedimentation mediated by 10% sucrose).
Fig.10-2 Distribution of the cell samples of marine sponge collected from South China Sea after density gradient centrifugation.
Fig.10-3 Cell samples of marine sponge collected from South China Sea harvested by each step of the method described in the invention (the cells at the bottom after velocity sedimentation mediated by 10% sucrose).
Fig.10-4 Dynamic curve of cell growth during primary culture in vitro between the purified archaeocytes and mixed cell population of the marine sponge collected from South China Sea.
Fig.11 Dynamic curve of cell growth in terms of MTT value during primary culture of the mixed sponge cells in comparative example.
Fig.12-1 Mold contamination during mixed sponge cell culture in vitro (hyphae enwinding among the sponge cell aggregation in comparative example.
Fig.12-2 Bacteria contamination during mixed sponge cell culture in vitro in comparative example. (Bacteria and Protozoa have shown in blue and purple after absorbing MTT, on the contrary the sponge cells which didn't absorb MTT showed their original yellow color).

### DESCRIPTION OF THE INVENTION IN DETAIL

### Example 1

In this example, marine sponge *Hymeniacidon perleve* was collected from Huang-bo sea in China. After being pretreated, the sponge was dissociated, and the multipotential stem cell of sponge, i.e. the archaeocytes, were purified effectively through the coupled four-step method. The purified archaeocytes were cultured in vitro, and metabolism of the bioactive compound was studied as follows.

### 1) Pretreatment (marine sponge dissociation, i.e. mixed sponge cells preparation)

Healthy sponge specimens in weight of 2~3g were soaked and washed for 3 times in sterile CMFSW (Ca²⁺/Mg²⁺-free seawater, 460 mM NaCl, 7mM Na₂SO₄, 10 mM KCI, 10mM Hepes, pH 8.0) supplemented with gentamicin(400µg/ml) to delete the bateria and other commensal. The sponge specimens were cut into small pieces (1-2 cm³) and stored in sterile CMFSW at 4°C for 8h, then treated with CMFSW having 25ppm CuSO₄ for 3hours. The small sponge pieces were washed with CMFSW for 3times, 2 hours each time to delete CuSO₄. The small sponge pieces were dissociated by rotating at 160rpm, and the resultant cell suspension was filtered through a 300mesh sieve, centrifuged at 1000rpm for 10min, the pellets were resuspended in sterile CMFSW with 2 mM EDTA to prevent the cells aggregating(1m 3mg/ml lysozyme in 30ml EDTA). Hence, mixed sponge cells were prepared (see fig. 1-1). The archaeocyte percent in the mix sponge cells was detected by a haemacytometer, and the BrdU (see fig. 1-2) and PCNA (see fig. 1-3) positive cells were also detected.

### 2. Archaeocytes purification

### 2.1 Differential centrifugation-enrich big sponge cells including archaeocytes

Adjust the density of the mixed sponge cells obtained by step 1) to a density of 5×10⁷/ml, then centrifugated at 300 rpm for 15min, discarded the supernant, the pellets were mostly big sponge and archaeocytes were included (see fig. 1-4). The archaeocyte percent, the BrdU (see fig. 1-5) and PCNA (see fig. 1-6) positive cells were detected. The rest cells are for further centrifugation.

### 2.2 Differential aggregation-enrich sponge cells apt to aggregate(including archaeocytes, pinacocytes and collencytes)

The sponge cells obtained by step2.1 were washed with CMFSW for twice to discard residual EDTA, and resuspended in CMFSW at a density of 10×10⁷/ml. Then a volume of 10ml of the sponge cells were cultured in a 100ml flask at 18°C for 8 h, rotating at 20rpm allowing the archaeocytes to aggregate as many as possible. The aggregates (see fig. 2-1)were collected by decanting the non-aggregating suspension cells. The archaeocyte percent in the aggregation (see fig. 2-7), the BrdU (see fig. 2-8) and PCNA (see fig. 2-9) positive cells were detected. The rest cells are for further centrifugation.

### 2.3 Differential adhesion-to separate the archaeocyte from collencytes

Collected aggregates were cultured in ASW (Artificial Sea Water, 460 mM NaCl, 7mM Na₂SO₄, 10 mM KCl, 10 mM CaCl₂, 50 mM MgCl₂, 10mM Hepes, pH 8.0)for 8 hours to adhere to the substrate. The pinacocytes and collencytes in the aggregates started to adhere to the bottom of culture flasks or dishes, these two cell types always distribute in the base of the adhering aggregate. These cells flattened and spread actively, once the cell confluence was reached 80% (see fig. 2-5), the ASW was washed with CMFSW and the archaeocytes on the top of the adhering aggregate were detached with CMFSW containing 10 mM EDTA (see fig. 2-6). The detaching time was optimized under light microscopy to prevent the pinacocytes and collencytes peeling off. This detaching process lasted for 5 min at 22°C. To reduce the negative chemical effect to sponge cells, the concentration of the EDTA we used is 2mM. Based on the adhering pattern of non-archaeocyte, this step removed most pinacocytes and collencytes from the aggregates (see fig. 2-10). After this step, briefly purifed archaeocytes were obtained, i.e. archaeocytes were separated from pinacocytes and collencytes. The archaeocyte percent, the BrdU (see fig. 2-11) and PCNA (see fig. 2-12) positive cells were detected. The detached archaeocytes cells were completely dissociated for the final step of density gradient centrifugation.

### 2.4 Density gradient centrifugation-- further separate archaeocyte from other cell types

1) adjust the sponge cells obtained by step 2.3 to density of 10×10⁷/ml
2) preparing gradient solution
   Solutions of 3% Ficoll (F) and 38% Vrografin (V) were prepared in sterile CMFSW at a density of 1.0276g/ml and 1.1486 g/ml at 16°C respectively. Continuous Ficoll-Vrografin (FV) gradients with densities of 1.04/1.05/1.06/1.07/1.08/1.09/1.10 g/ml were developed (see fig. 3-1).
3) Density gradient centrifugation
   The volume of the tube we used is 15ml, and the volume of each sucrose gradient solution is 1 ml, and 1ml dissociated enriched archaeocyte suspension was applied on the top of the sucrose gradient, total cell number being treated in this step is 2×10⁷. Then the cells were centrifuged with a horizontal centrifuge at 1000rpm for 20min for further separating archaeocyte from other cell types.
   After centrifugation, the cell fractions that accumulated at interfaces between successive densities were collected individually by a Pasteur pipette and washed with CMFSW twice to remove Ficoll and Vrografin. Cell fractions was observed under light microscopy to identify the types of cells and their relative numbers and morphology, interface between density 1.07 and 1.08 (see fig. 3-2), the archaeocyte percent(see fig. 4-1), the BrdU (see fig. 4-2) and PCNA (see fig. 4-3) positive cells were also detected.

### 3. Detection of the proliferation potential of purified archaeocytes

### 1) BrdU labelling assay and immunocytochemical assays

Sampling from the sponge cells harvested from each step of the combined method referenced above, the cell density and archaeocyte content were counted. Add BrdU labeling medium (1: 1000) to the cells at a ratio of 0.5ml BrdU to 10⁶ cells. The cells were incubated at 18°C for 24h. The BrdU positive cells were detected by using a BrdU labeling and detection kit following the manufacturer's instruction (Roche).
At the same time, Effect of incubation time on the incorporation of BrdU into the Archaeocytes was also examined (see fig. 6)

### 2) PCNA expression

PCNA assay was used to study the proliferation capability of sponge archaeocytes. The results showed that during the purification process, the percentage of PCNA-positive cells increased gradually and correlated closely with the purity of the archaeocytes (see Fig. 5). This showed that the sponge cells purified had high capability of proliferation, proving that the cells we purified were archaeocytes,

### 4. Sponge cell culture in vitro and metabolism examing

As marine sponge *Hymeniacidon perleve* for example; the mixed sponge cells were inoculated at a density of 8×10⁶/ml, in sterile MASW(Modified Artificial Sea Water). The culture media was changed by 50 percent every 2 days. The cultures were maintained at 18°C in an incubator, at rest or rotating, and fluorescent lamp was used.

Modified Artificial Sea Water is composed of basic artificial sea water and intercellular substance of the sponge.

Component of the artificial sea water is as follows: 460 mM NaCl, 7mM Na₂SO₄, 10 mM KCl, 10 mM CaCl₂, 50 mM MgCl₂ , 20mM Tris, 30mM Na2SiO3.9H2O, 60µM FeC₆H₅O₇.5H₂O, 4µM ZnCl₂, 5µM Vit C, 10µM L-Glutamine, 10µM L-asparagin, 10µM glycine, 20µM glycose, 1mM sodium pyruvate, pH 8.0.

The described intercellular substance of sponge is prepared as follows: 10g freshly collected sponges were dipped in 100ml CMFSW. Due to the lack of Ca⁺⁺, sponge tissues are rarefaction and the intercellular substance will be released into CMFSW. The CMFSW was changed by 90percent every 2hours, totally 5times. 1 L lixivium was collected and filter-sterilized by 0.22-µm membrane to obtain lixivium for cell culture. The extracted intercellular substance was added to the cell culture system with a ratio of 1:1.

The archaeocytes purified at step 1 were cultured in MASW and cell proliferation and metabolism were detected, comparing with those in mixed-cell group. The cell growth of the archaeocytes and mix sponge cell were showed in fig. 7 and fig. 8. The production of β-carotene could reach 0.01 mg/ (10⁶ cells/ml), and the production of 5α-cholesterol-3β-steroid is 0.1mg/5x10⁶ cells/ml. The results have shown that the bioactive compounds could be produced in a large scale by sponge cell culture in vitro.

### Example 2

Purification of the archaeocytes in a marine sponge collected from South China Sea (species was not identified)

The basic principle and method of the purification process is same with example 1. Detailed process is modified because of the difference of sedimentation coefficient and aggregation capability between different species. The detailed purification process which differs from the example 1 is as follows:
Step 1: Sponge specimens which were kept in a aquarium for 2 weeks were soaked in sterile NSW (natural seawater) supplemented with gentamicin(800µg/ml). The bacteria and other protozoan were washed away. Sponge specimens were cut into small pieces (2-3 mm³) and rinsed in sterile CMFSW (986mM NaCl, other components are the same with the one described in example 1) for 4 times, 3hours each time. The small sponge pieces were dissociated with CMFSW-E, and the resultant cell suspension was filtered after rotation and centrifuged at 2000rpm for 10min, the pellets were resuspended in sterile CMFSW with 2 mM EDTA to prevent the cells aggregating. Hence, mix sponge cells were prepared.
Step 2.1: adjust the mix sponge cell to a density of 2×10⁷/ml (see Fig. 9-1), then centrifuged at 500rpm for 10min, the pellets are bigger cells from cortex and medulla of the sponge (see Fig. 9-2).
Step 2.2: adjust the cells harvest at step 2.1 to a density of 5×10⁷/ml with CMFSW, and cultured in flask at 25°C for 12hours, rotating. Discard the non-aggregating cells, the cell aggregations (see Fig. 9-3) were prepared for next step.
Step 2.3: The cell aggregation were continually cultured in ASW (986mM NaCl, other components are the same with the one described in example 1)for 10 hours to adhere to the substrate. The pinacocytes and collencytes in the aggregates adhered, spread actively.Once the cell confluence reached 80%, the ASW was washed with CMFSW and the archaeocytes on the top of the adhering aggregate were detached with CMFSW containing 5 mM EDTA (see fig. 9-4). The detaching process was optimized under light microscopy lasting for 10 min at 18°C (when 10 mM EDTA was used, the detaching time was 5 min). The detached archaeocytes cells were completely dissociated for the final step of density gradient centrifugation.
Step 2.4
   1) adjust the sponge cells harvested at step 2.3 to a density of 5×10⁷/ml
   2) The sponge cells harvested at step 2.1 was treated by velocity sedimentation with low concentration sucrose (we use 10% sucrose in this example).The cells were separated into three fractions according to velocity of sedimentation (see fig. 10-1). Cells on the top layer are small cortex cells, the layer in the middle is composed of bigger cells from cortex and medulla besides some archaeocytes (archaeocytes accounted for almost 10%). The lowest layer, which is the fastest in velocity of sedimentation, is archaeocytes and other big cells (archaeocytes accounted for almost 65%) (see fig. 10-3 for the fastest part in velocity of sedimentation at step 2.2). Then the lowest part was treated by sucrose density gradient centrifugation. The gradients were developed by gentle layering successively less-dense sucrose solutions (20%, 30%, 40%, 50%, 60%) (see fig. 10-2). The archaeocytes distributed at the lowest layer of highest cell density with a purity over 80%. We could also purify another type of sponge cells using the above method, but the said cell type was unknown (see fig.9-5).
   3) when a 50ml tube was used for density gradient centrifugation, volume of each gradient was 8ml, and the cell suspension applied was 5ml, total cell number to be treated was 1×10⁸. The harvest archaeocytes were washed with CMFSW to delete gradient solution using a horizontal centrifuge at 1500rpm for 10min for further separation.
Step 3: The archaeocytes harvested at step 1 were adjust to a density of 2×10⁶ cells/ml for further culture, and were inoculated in sterile MASW (Modified Artificial Sea Water). The cultures were maintained at 25°C in dark rotating at 40 rpm. The culture media was changed by 70 percent every other day. Cell numbers were monitored to compare the cell proliferation with that in the mixed cell group.
The described MASW (Modified Artificial Sea Water) is composed of basic artificial sea water and factors that promoting sponge cells to grow: 60 µg/L Na₂SeO₃, 1mg/L DTT, 240 µg/L GSH
The cell growth dynamic curve of the purified archaeocytes and mix sponge cells were showed in fig. 10-4.

### Example 3

Purification of archaeocytes from a marine sponge collected from South China Sea (species was not identified)

This example was different from the example 2 as follows:
Step1:Sponge specimens which were kept in a aquarium for 7 days were soaked in sterile NSW (natural seawater) supplemented with gentamicin (200µg/ml) to wash away the bacteria and other protozoan. Sponge specimens were cut into small pieces (1-3 mm³) and rinsed in sterile CMFSW (986mM NaCl, other components are the same with the one described in example 1) for 5 times, 2.5 hours each time. The small sponge pieces were dissociated with CMFSW-E, and the resultant cell suspension was filtered and centrifuged at 2500rpm for 20min, the pellets were resuspended in sterile CMFSW with 3 mM EDTA. Hence, mix sponge cells were prepared.
Step 2.1: adjust the mix sponge cell harvested at step 1 to a fitful density, then centrifuged at 600rpm for 20min, the pellets obtained by enrichment are bigger cells from cortex and medulla of the sponge.
Step 2.2: adjust the cells harvest at step 2.1 to a density of 7×10⁷/ml with CMFSW, and cultured in flask at 20°C for 10hours, rotating. Discard the non-aggregating cells, the cell aggregations were prepared for next step.
The cell aggregation were cultured in ASW (986mM NaCl, other components are the same with the one described in example 1) for 10 hours to adhere to the substrate. The pinacocytes and collencytes in the aggregates adhered, spread actively.Once the cell confluence reached 75%, the ASW was washed with CMFSW and then the archaeocytes on the top of the adhering aggregate were detached with CMFSW containing 6 mM EDTA. The detaching process was optimized under light microscopy lasting for 5 min at 20°C (when 5 mM EDTA was used, the detaching time was 7 min). The detached archaeocytes were dissociated completely for further density gradient centrifugation.
Step 2.4:
   1) The sponge cells harvested at step 2.3 was adjusted to a density of 5×10⁷/ml
   2) The sponge cells harvested at step 2.1 was treated by velocity sedimentation with low concentration sucrose (20% sucrose in this example).Then followed by the sucrose density gradient centrifugation. The gradients were developed by gentle layering successively less-dense sucrose solutions (12%, 24%, 36%, 48%, 60%).
   3) when a 50ml tube was used for density gradient centrifugation, volume of each gradient was 10ml, and the cell suspension applied was 3ml, total cell number to be treated was 2×10⁸. The harvest archaeocytes were washed with CMFSW for 4 times to delete gradient solution using a horizontal centrifuge at 500rpm for 15min.
Step 3: The archaeocytes were cultured at 25°C in dark.
   The described MASW is composed of basic artificial sea water and differentiation-controlling factors: 20µM/ml hydroxyacrbamide, 10µM/ml retinoic acid

The archaeocytes purified at step 2.4 were cultured at a density of 3×10⁷ cells/ml. The cultures were maintained at 25°C in dark rotating at 20 rpm. The culture media was changed by 70 percent every other day. Cell numbers were monitored to compare the cell proliferation with that in the mixed cell group.

This invention introduced for the first time the stem cell culture of marine sponge. We have successfully established a novel four-step coupling protocol for the selective purification of sponge archaeocytes (stem cells). The rationale for this protocol is based upon the multipotent and proliferation properties of archaeocytes, with the method in the invention progressively achieving a high purity of archaeocytes. Based on this purification method, the invention also established the technology condition of the stem cell culture in vitro, including physical and chemical condition, media, key factors modulating differentiation and growth, culture protocol and bioreactor and other key technique. The stem cell cultured in vitro could proliferate steady and produce bioactive compounds. This invention provided a novel way to produce key drug-source sponge biomass and bioactive compounds. It is very significative for the industrialization of marine drug, especially sponge drug. This technology will present a wide commercial foreground. Moreover, It will also provide a crucial tool for the cell biology research of marine invertebrate. So, this invention has a important theory meaning and application value.

The described MASW in this invention based on artificial sea water, adding factors that promoting sponge cells to grow using any one selected from the group consisting of soluble cholesterol, hydrocortisone, insulin, transferring, phytohaemagglutinin (PHA), and mixtures thereof, these factors could be used solely or combined randomly; the danddifferentiation-controlling factors such as hydroxyacrbamide and retinoic acid, they could also be used solely or combined together.

### Comparative example

### Cell growth in mix sponge cell culture with ordinary method

Mix sponge cells were harvested by ordinary chemical dissociation method, and aggregated in ASW or NSW. To prevent microorganism contamination, 100 U/ml gentamicin, 100U/ml penicillin, 100µg/ml streptomycin, 3µg/ml Amphotericin were used in the culture system. Cell growth was detected by MTT incorporation assay, the results see fig. 11. It showed MTT value began to increase on the fourth day, the maximus cell growth was more than eightfold on the seventh day. But a mass of bacteria and mold were discovered under microcope. So the rapid increase of the MTT value owned to the growth of bacteria and mold, not sponge cell growth (see Fig. 12-1, 12-2). The maximus biomass detected on the fourth day owned to the contamination of protozoan. The results suggested that the contamination problem badly disturbed the cell growth in the mix sponge cell culture produced by ordinary cell dissociation method.

## Claims

1. A purification and cultivation method of multipotential stem cell of marine sponge, **characterized in that** the pretreated mixed sponge cells were progressively treated by differential centrifugation, differential aggregation, differential adherence and density gradient centrifugation, purification carries out according to the properties of sponge cells of size, adhesion:-
1) Pretreatment namely preparation of mixed sponge cells:
Healthy fresh sponge specimens or marine sponge specimens were kept in a aquarium for7~14days before use were soaked in sterile natural seawater supplemented with gentamicin of 200~800µg/ml to delete the impurity on the sponge surface; Sponge specimens were cut into 1~3mm³ small pieces and rinsed in sterile Ca²⁺/Mg²⁺-free seawater; The small sponge pieces were dissociated and the resultant cell suspension was filtered and centrifuged at 1000~3000rpm for 10~20min, the pellets were resuspended in sterile Ca²⁺/Mg²⁺-free seawater with 1~2 mM EDTA to prevent the cells aggregating; Hence, mixed sponge cells were prepared;
2) Purification of multipotential stem cell of marine sponge:
2.1 Differential centrifugation:
Adjust the density of the mixed sponge cells of the step 1) with Ca²⁺/Mg²⁺-free seawater to a fitful value, then centrifugated at 300∼600 rpm for 10~20min, discarded the supernant, the pellets were mostly big sponge and archaeocytes were included;
2.2 Differential aggregation:
The sponge cells obtained by differential centrifugation as above the step 2.1 were washed with Ca²⁺/Mg²⁺-free seawater to remove EDTA, and resuspended in Ca²⁺/Mg²⁺-free seawater; Then the sponge cells were cultured at 18~25°C for 8~12 h allowing the archaeocytes to agglomerate; The aggregates were collected by decanting the non-aggregating suspension cells;
2.3 Differential adhesion:
The collected aggregates were continuously cultured in artificial sea water to adhere to the substrate; The pinacocytes and collencytes in the aggregates started to adhere to the bottom of culture flasks or dishes; These cells then flattened and spread actively, once the cell confluence was reached 70~80%, the artificial sea water was washed off with Ca²⁺/Mg²⁺-free seawater and the archaeocytes were dissociated with Ca²⁺/Mg²⁺-free seawater containing 2~10 mM EDTA; The dissociation time was optimized by regular inspection under light microscopy to prevent the release of under-stratum cells; In general, this process lasted for 5-10 min at 18-22°C; Based on the adhering pattern of non-archaeocyte, this step removed most pinacocytes and collencytes from the aggregates; i.e. the detached archaeocytes cells were completely dissociated in CMFSW-E for the final step of density gradient centrifugation;
2.4 Density gradient centrifugation
2.4.1 The sponge cells harvested at step 2.3 were adjusted to a density of (5-10)×10⁷/ml for density gradient centrifugation;
2.4.2 Prepare the density gradient solution;
2.4.3 The cells preliminarily dissociated were density gradient centrifuged after the concentration was adjusted;
Continuous gradients were developed layering of 1-10 ml successively density gradient; Then, 1~5 ml dissociated enriched archaeocyte suspension, total cell number is 2~20x10⁷, was applied on the top of the gradient. After centrifugation, the archaeocyte fraction were collected and washed with Ca²⁺/Mg²⁺-free seawater by centrifugation at 500~1500rpm for 5-20min to remove gradient solution. This washing process was carried out for 2-4 times, thus the harvest archaecytes could further be separated from other cell types.

2. The method according to the claim 1, **characterized in that** wherein the fitful concentration of EDTA as described at the step 2.3 in the Ca²⁺/Mg²⁺-free seawater for detaching the archaeocytes on the top of the adhering cell aggregate was 2~5mM.

3. The method according to the claim 1, **characterized in that** wherein density gradient described in step 2.4 was prepared as follows: Solutions of 3-9% Ficoll were prepared in sterile Ca²⁺/Mg²⁺-free seawater at a density of 1.0276~1.0445 g/ml at 16~20°C, and 34-38% Vrografin were prepared with 76% vrographin in sterile Ca²⁺/Mg²⁺-free seawater at a density of 1.1379~1.1486 g/ml at 16~20°C, respectively; Continuous Ficoll-Vrografin gradients with densities of 1.04/1.05/1.06/1.07/1.08/1.09/1.10 g/ml were developed.

4. The method according to the claim 1, wherein density gradient described in step 2.4 could also be prepared with sucrose as follows: sucrose solutions were prepared at a concentration of 12~24%, 24~36%, 36~48%, 48~52%, 58~60%.

5. The method according to the claim 1, **characterized in that** wherein as described at the step 2.4, the volume of each gradient solution is 1~2 ml when the volume of the used tube is 15ml, and 1~2 ml dissociated enriched archaeocyte suspension was applied on the top of the gradient; The volume of each gradient solution is 5~10 ml when using 50ml tube, and 3∼5 ml dissociated enriched archaeocyte suspension was applied, the cells were totally 1~2×10⁸.

6. The method according to the claim 1, **characterized in that** wherein the sponge cell culture in vitro and metabolism detection were added in this invention as follows: the purified multipotent archaeocytes harvested at the step 1 were cultured in a density of 2×10⁶~3×10⁷ cells/ml with artificial sea water at 18~25°C, at rest or rotating at 20~40rpm; the culture media namely modified artificial sea water was changed by 50-70 percent every 1-2days; The total cell number, archaeocytes number and PCNA positive cell number were counted; At the same time, mixed sponge cell culture was also carried out as control to compare cell proliferation;
Wherein the component of the modified artificial sea water used for cell culture is: artificial sea water added with intercellular substance of the sponge, cell growth factors or differentiation-controlling factors.

7. The method according to the claim 6, wherein the cells could be cultured in light or dark according to the sponge species.

8. The method according to the claim 6, **characterized in that** wherein the described intercellular substance of sponge is prepared as follows, freshly collected sponges were dipped in Ca²⁺/Mg²⁺-free seawater in ration of 1/10~1/20 (w/v); The Ca²⁺/Mg²⁺-free seawater was changed by 80-100percent every 2-4hours, totally 4-6times; All the lixivium collected and sterilized through 0.22-µm; The extracted intercellular substance was added to the cell culture system by 25-75persent.

9. The method according to claim 6, **characterized in that** wherein the described cell growth promoting factors as follows: 5-20mg/ml soluble cholesterol, 5-20µg/ml hydrocortisone, 5-10µg/ml insulin ,5-10µg/ml transferrin, 6-10×10⁻⁵g/L Na₂SeO₃, 15-50µg/ml phytohaemagglutinin (PHA), 100-200µg/ml DTT(1,4-Dithiothreitol), 2-8×10⁻⁴g/L glutathione, these factors could be used solely or combined randomly.

10. The method according to the claim 6, **characterized in that** wherein the described differentiation-controlling factors are: 5-50µM/ml hydroxyacrbamide, 5-20µM/ml retinoic acid, they could be used solely or combined randomly.
